# EUROPEAN PATENT SPECIFICATION

(11) **EP 3 323 146 B1**
(45) Date of publication and mention of the grant of the patent: **28.02.2024**
(21) Application number: 16757271.8
(22) Date of filing: 14.07.2016
(51) Int. Cl.: A61L 2/10, A61L 2/24, A61L 9/20, A61N 5/06, H01L 25/075, H01L 33/50, F21V 33/00, H05B 45/10, F21Y 113/13

(54) **USE OF A LIGHT EMITTING DIODE BASED LIGHTING DEVICE FOR DISINFECTION**
VERWENDUNG EINER LEUCHTDIODENBASIERTEN BELEUCHTUNGSVORRICHTUNG ZUR DESINFEKTION
UTILISATION D'UN DISPOSITIF D'ÉCLAIRAGE À BASE DE DIODES ÉLECTROLUMINESCENTES POUR LA DÉSINFECTION

(30) Priority: 14.07.2015 US 201514798496
(43) Date of publication of application: 23.05.2018
(73) Proprietor: CalyxPure, Inc., Houston, TX 77014 (US)
(72) Inventor: RANTALA, Juha, 8806 Bäch (CH)
(74) Representative: Laine IP Oy
(86) International application number: PCT/FI2016/050518
(87) International publication number: WO 2017/009534

(56) References cited:
- EP-A1- 2 211 083
- WO-A1-2010/053341
- CN-U- 201 935 074
- JP-A- 2005 101 458
- US-A1- 2009 224 652
- US-A1- 2009 323 306
- US-A1- 2011 058 372
- US-A1- 2011 085 936
- US-A1- 2012 043 552
- US-A1- 2015 014 715
- M. MACLEAN ET AL: "Inactivation of Bacterial Pathogens following Exposure to Light from a 405-Nanometer Light-Emitting Diode Array", APPLIED AND ENVIRONMENTAL MICROBIOLOGY, vol. 75, no. 7, 1 April 2009 (2009-04-01), pages 1932-1937, XP055307098, US ISSN: 0099-2240, DOI: 10.1128/AEM.01892-08
- SHUR M S ET AL: "Deep-Ultraviolet Light-Emitting Diodes", IEEE TRANSACTIONS ON ELECTRON DEVICES, IEEE SERVICE CENTER, PISACATAWAY, NJ, US, vol. 57, no. 1, 1 January 2010 (2010-01-01), pages 12-25, XP011285139, ISSN: 0018-9383, DOI: 10.1109/TED.2009.2033768

## Description

### FIELD OF INVENTION

The present invention relates to use of artificial lighting arrangements for disinfection by light. In particular, the present invention relates to the field of optoelectronics and white light emitting diodes (LEDs) providing bactericidal effects. The present invention relates to the use of an integrated LED structure in a continuously operating disinfection luminaire.

### BACKGROUND ART

It is well known that ultra-violet (UV) sources have bactericidal and fungicidal effects, well suited for disinfection. Deep UV (UVC) sources are known to effectively prevent bacterial growth on surfaces and are widely used as germicidal sources. However, the drawback of usage of UVC sources such as mercury lamps is the fact that UVC light is harmful for humans and thus prevents its use in presence of people. The mechanism behind the deep UV disinfection is known to be the cracking of DNA molecules, which have particularly strong absorption between 260 - 290nm.

It is also known that longer wavelengths produce also bactericidal effects, although based on different physical mechanism. UVA light at 365 nm is known to inhibit bacterial growth and also blue/violet light produces similar growth blocking effects. Although the bactericidal effect is less strong at blue/violet wavelengths it can be exploited in continuously operating disinfection lights. The 405 nm light is well known to cause reactive oxygen species **(ROS)** generation in cells. These negatively charged oxygen ions in turn prevent cell metabolism and effectively suppress e.g. growth of bacterial colonies. While the intensity of the disinfection light is of primary importance, it is the total dose, expressed in terms of J /m², accumulated on the surface or on the objects, which ultimately define the disinfection power.

Any lower intensity source with suitable emission spectrum can be used for disinfection as long as the exposure times are long enough but still of practical value. However, again the human presence sets boundaries for such lights. International regulations and safety guidelines are defined by the International Commission on Non-Ionizing Radiation Protection (ICNIRP) and IEC standard IEC-62471. Again, the ICNIRP defines ultra-violet wavelengths to be 100 - 400 nm.

If the radiation source has a short wavelength emission, say below 410 nm, and this short wavelength emission is the dominating intensity or color, humans are commonly experiencing discomfort.

Known growth lights for plant growth and photosynthesis comprise blue and red light sources that are sometimes accompanied with white light sources. Hence they are not addressing issues relating to antibacterial and fungicidal function.

A light source applying LEDs and UV germicidal lamp is disclosed in CN 104056289 A. However, again such assembly is not suitable for general lighting due to detrimental effects of UV light to humans.

A LED source with disinfection capability in closed environment is presented in EP 2 554 583 A1. Again such source emitting wavelengths below 300 nm is not suitable for general lighting due to detrimental effects of UV light to humans.

According to our laboratory tests a combination of individual, spatially separated LEDs with 405 nm emission and individual white light LEDs results in a light source that causes discomfort. A light source based on individually packaged LEDs does not produce a smooth and uniform light field. Particularly point sources with intensive short wavelength emission are disturbing. It is necessary to provide a source in which the short wavelength point sources are not distinctively visually appearing between the white light LEDs. However, physical overlaying of the white light LEDs with 405 nm LEDs is not straightforward or possible.

An example of spatially mixing the source is shown in US 8,398,264 B2. A diffuser plate is used in conjunction with Fresnel type lens to provide uniform emissions and to avoid direct visibility of individual short wavelength emitters in the source plane. The known diffuser based constellation is complex and expensive.

US 2015/0014715 A1 discloses a white light LED module structure including ultraviolet light. An ultraviolet light LED chip is disposed in a packaging structure of the white light LED module. The ultraviolet light LED chip radiates an ultraviolet light. The white light and the ultraviolet light simultaneously illuminate an object.

JP 2005-101458 A discloses a semiconductor light emitting device, which includes at least two kinds of light emitting elements in a light emitting element having a function of emitting light effective for sterilization, a light emitting element having a function of emitting light effective for ozone generation, a light emitting element having a function of emitting light effective for a photo-catalyst, and a light emitting element having a function of emitting illumination light or emitting illumination light by emitting a fluorescent body. The light emitting elements are connected in parallel with each other.

US 2009/0224652 A1 discloses white light illumination systems (so called "white LEDs") that comprise a multi-chip excitation source and a phosphor package. In a two-chip source, the two LEDs may be UV emitting and blue emitting, or blue-emitting and green-emitting. The phosphor package is configured to emit photo luminescence.

US 2012/0043552 A1 discloses an LED pump light with multiple phosphors. LEDs emitting radiation at violet and/or ultraviolet wavelengths are used to pump phosphor materials that emit other colors. The LEDs operating in different wavelength ranges are arranged to reduce light re-absorption and improve light output efficiency.

EP 2 211 083 A1 discloses an illuminating device comprising semiconductor light-emitting devices employing a semiconductor light-emitting element, wherein outputted light is stably combined, separation of light is inhibited, and color tone is variable.

US 2009/0323306 A1 discloses a conversion type light emitting device, which includes at least one LED element having a predetermined light emitting range and a fluorescent element performing wavelength conversion of light emitted from the LED element when disposed at a first location in the predetermined light emitting range. The fluorescent element is movable from a first location to a second location outside the predetermined light emitting range.

WO 2010/053341 A1 discloses a phosphor conversion light-emitting diode for meeting photomorphogenetic needs of plants. The plant-cultivation LED with conversion of light in phophors contains one type of light emitting source.

### SUMMARY OF INVENTION

To solve above discussed problems it is an object of the present invention to provide a use, in accordance with the appended claim 1, of a lighting device for disinfection.

Further, advantageous embodiments of the present invention are the object of the appended dependent claims.

The present invention provides a use of a lighting device for disinfection comprising at least one LED structure comprised of
a substrate;
a light emitting area defined on the substrate as a cavity;
a first type of light emitting semiconductor source with bactericidal and germicidal characteristics mounted in the cavity;
a second type of light emitting semiconductor source mounted in the cavity with ability to excite a wavelength conversion material to generate white light; and
the wavelength conversion material formed as a layer on top of the light emitting semiconductor sources, wherein
- in the presence of humans, the first type of light emitting semiconductor source is adjusted to provide white light illumination with a bactericidal and germicidal low-intensity emission in the background of the white light emission, non-perceptible to human eye; and
- in a situation with no humans present, the white light illumination generated by the second type of light emitting semiconductor source exciting the wavelength conversion material is turned off, while the bactericidal and germicidal emission is maximized.

In accordance with the present invention as claimed, there is wavelength conversion material layer formed on top of the light emitting semiconductor sources, and typically an electrical circuit layer for connecting the light emitting semiconductor sources to electrical control interface.

Considerable advantages are obtained.

Thus, the present invention provides use of a lighting device which achieves a continuous disinfection process.

The invention enables disinfection by a lighting device visibly apparent to human as a white light source that is neither harmful to humans nor creates discomfort. This aim is achieved by overlaying the short wavelength emission with white light emission. The use of such a lighting device for disinfection in accordance with the appended claims is suitable for the general illumination purposes while simultaneously providing means to disinfect exposed surfaces and objects. The said light emitting area comprises several LED semiconductor diodes, which are technically reliable and economically viable, as light emitters to provide the light emission.

The use of electromagnetic radiation at wavelength of 405 nm as disclosed in this invention is safe. The new type of LED sources disclosed avoids the disturbing effect of the shortwavelength visible light.

The present invention provides disinfection functionality for general lighting enabled by the disclosed integrated LED structure and luminaire.

Spatial integration gives an integrated LED structure which has, advantageously, both 405 nm emission source and a 450 nm blue emission source buried under a wavelength conversion layer, in very near vicinity of each other. In a preferred embodiment, independent control of the two emissions, namely the 405 nm radiation and the white light emission, is provided for.

This allows for the control of intensity of 405 nm radiation so that in situations where no white light is needed, such as when no human is present in accordance with the present invention as claimed, only 405 nm radiation is to be used and with maximum intensity, while white light radiation is to be turned off. And conversely when humans in accordance with the present invention as claimed, or in some applications - not forming part of the present invention as claimed-animals, are present the radiation intensity of 405 nm is to be turned low, or in applications not forming part of the present invention as claimed completely off, while maintaining suitable level of white light illumination

With the integrated **LED** structure being used in accordance with the appended claims, the white light quality parameters, such as **CRI** and CCT. remain constant as the 405 nm emission has negligible contribution to the luminous flux or illuminance.

The integrated LED structure being used in accordance with the appended claims provides means for spatially mounting the emitter with bactericidal and germicidal effect in close vicinity of the white light source. When, in the presence of humans in accordance with the present invention as claimed, the intensity of the bactericidal short wavelength is appropriately chosen with respect to the white light intensity, the source appears as a normal white light source to human eye. Furthermore the spatial arrangement guarantees that the emission of 405 nm is not distinguishable for humans due to overlaying white lightemission.

### BRIEF DESCRIPTION OF DRAWINGS

The invention will now be further described, by way of non-limiting examples, with reference to the accompanying diagrammatic drawings. In the drawings:

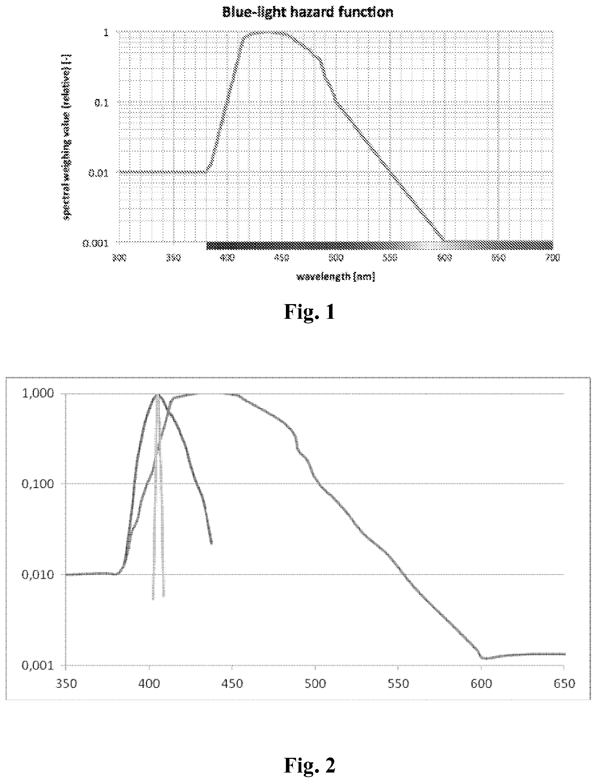

- Figure 1: Is a graph presenting spectral weighting function of blue-hazard light.
- Figure 2: Is a graph presenting spectral weighting function of blue-hazard light with typical emission spectrum of light emitting diode at 405 nm and a typical emission spectrum of a laser diode at 405 nm.

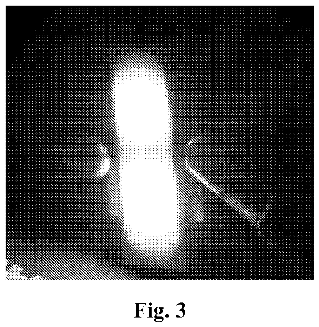

- Figure 3: Is a photo of LED source for continuous disinfection luminaire with spatially combined white light emitter and low wavelength emitter in one LED source and the spectrum is spatially combined.
- Figure 4: Is a schematic top side view of an integrated LED structure to be used in accordance with an embodiment of the present invention.
- Figure 5: Is a schematic view of the cross-section of an integrated LED structure to be used in accordance with an embodiment of the present invention.
- Figure 6: Is a graph representing a typical emission spectrum of an integrated LED structure to be used in accordance with an embodiment of the present invention.
- Figure 7: Is a graph representing typical emission spectra of an integrated LED structure to be used in accordance with an embodiment of the present invention.
- Figure 8: Is a schematic top side view of an integrated LED structure according to an embodiment not forming part of the present invention.
- Figure 9: Is a schematic view of the cross-section of an integrated LED structure according to an embodiment not forming part of of the present invention
- Figure 10: Is a graph representing a typical emission spectrum of an integrated LED structure according to an embodiment not forming part of the present invention

### DESCRIPTION OF EMBODIMENTS

The following descriptions are merely non-limiting examples, some of which are in accordance with the present invention as defined by the appended claims, whereas others are not in accordance with the present invention but are useful for illustrating its context. It will be appreciated by one skilled in the art that specific details of the examples may be changed without departing from the scope of the invention as defined by the claims.

The present technology provides an integrated LED structure and a luminaire for enabling, in accordance with the present invention as claimed, a continuous disinfection process.

In accordance with the present invention, disinfection is achieved by using a lighting device visibly apparent to human as a white light source that is firstly not harmful to a human and secondly is not creating discomfort. Such a white light source is suitable for the general illumination purposes while simultaneously providing means to disinfect exposed surfaces and objects.

The present technology also achieves a light source that provides for disinfection of objects and also for stimulated photosynthesis of plants. The disclosed integrated LED structure can be incorporated into white light source for providing disinfection with bactericidal and fungicidal, anti-viral (germicidal), anti-viral and, in addition, possibly also photosynthesis effects

Thus, disinfection functionality can be achieved for general lighting and photosynthesis lighting, enabled by the disclosed integrated LED structure and luminaire.

The emission area comprises wavelength conversion material to provide means for white light emission. Emission area comprises in some preferred embodiments more than one type of wavelength conversion materials. The materials can be layered vertically upon each other, or horizontally with different materials adjacent to each other, or in mixed material layers, to achieve high efficiency or high color rendering index (CRI), or wanted color correction temperature (CCT).

The light emitters are in some cases electrically connected in series or parallel to enable a common current drive scheme. The control interface has then at least one wire for providing the common drive current and least one ground wire to close the current path back to power supply. However, in some cases the emitters are not connected electrically together, thus enabling independent intensity control. The control interface has then at least three wires for providing the drive current independently, and at least one ground wire to close the current path back to power supply.

The integrated LED component, LED structure or package has two different types of semiconductor emitters, which can be also independent in terms of electrical circuits. In a nominal operation point the current is adjusted for both emitters simultaneously, however. with electrical circuitry being independent, current is adjusted separately and the emission appears as white light in both cases, but with the emission spectrum having a spectroscopically observable double peak structure with blue emission at or near 450 nm and violet emission at or near 405 nm.

If the intensity of the 405 nm emission is A and the intensity of the 450 nm emission is B, the ratio A/B can be now freely adjusted with the two independent drive currents. In nominal situation the ratio is adjusted so that the emission of 405 nm is distinguishable for human and is within the safety limits as discussed earlier. The source is emitting white light and at the same time giving low intensity emission at 405 nm to provide continuous disinfection functionality.

The intensity control is exploited dynamically depending of the human presence. In the first case of no human presence the ratio A/B can be maximized. In the second case of human presence the intensity A can be adjusted to a low value, and to comply with the safety standards. Thus there are at least two set points of operation in typical case. In the first set point of operation, the drive current is tuned up to e.g. 350 mA for the 405 nm emitter, while the drive current for the 450 nm emitter can be tuned down to 0 mA. In the second set point the drive current is tuned down to e.g. 50 mA for the 405 nm emitter, and the drive current for the 450 nm emitter is tuned to e.g. 350 mA. Thus while still maintaining the white light emission, the LED structure is providing illumination with bactericidal and germicidal effect. Such intensity tuning is beneficial for ensuring safety in presence of humans, and to avoid exposure to high intensity radiation at 405 nm.

In some preferred mode of use, the dynamic intensity tuning can be exploited to adjust the emission of 405 nm after a certain total radiation dose has accumulated on the target surface. This can be detected by integrating the specific wavelength signal with a detector circuit and providing the necessary feedback to control appropriately the output of the integrated LED structure. This is beneficial for reducing energy consumption and to lengthen the life time of the LED by avoiding unnecessary use of the 405 nm emitter.

The complete emission of the white light source is formed of as a sum of the emission of the 405 nm emitter and of the emission of the 450 nm emitter, and the emission from the wavelength conversion material, which is excited by the emission of the 450 nm emitter.

In some embodiments not forming part of the present invention as defined by the claims, the integrated LED structure comprises only one type of light emitter, preferably having a short wavelength emission below wavelength of 410 nm, and a wavelength conversion material layer formed on top of the emitter.

In such embodiments, not forming part of the present invention as defined by the claims, the complete white light source emission has the light emission spectrum formed of as a sum of emission from the 405 nm emitter and the emission from the wavelength conversion material, excited by the emission at 405 nm.

In preferred embodiments the wavelength conversion material is phosphor based e.g. in YAG:Ce materials providing white light emission spectrum with CRI and CCT characteristics suitable for general lighting applications. The wavelength conversion material has in this case a relatively low extinction coefficient at the wavelength range of 360 to 410 nm to avoid excessive absorption of the emission below 410 nm.

The said emission area can be formed as buried shallow cavity on the top surface of the said substrate. In some preferred embodiments the LED structure can comprise several emission areas in buried cavities of different heights.

The short wavelength emitter has an emission wavelength that has bactericidal, germicidal or fungicidal effects. In preferred embodiments the short wavelength emission or intensity has no or negligible detrimental effects to human skin, human eyes, or human health in general.

In some embodiments the short wavelength emitter has an emission wavelength that has bactericidal, germicidal or fungicidal effects and the emitter is also emitting at wavelengths to support, enhance and propagate photosynthesis in plants.

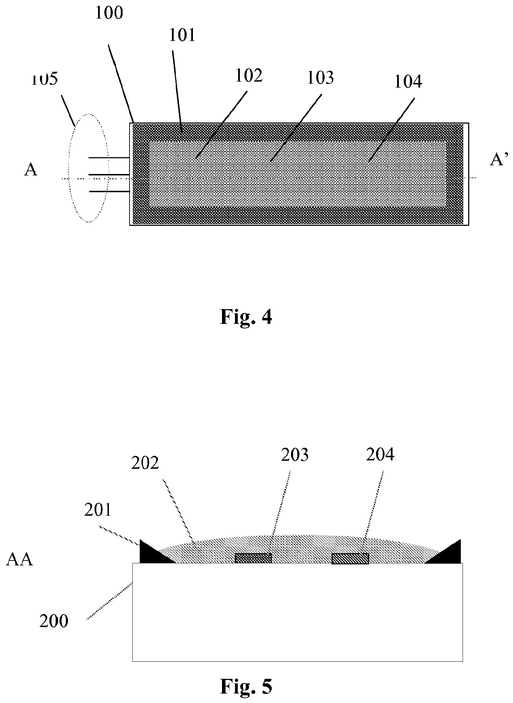

Turning now to the embodiments shown in the drawings, it can be noted that in one embodiment (yellow phosphor), the LED structure (Fig. 4) is comprised of a substrate 100, an emission area inside the cavity wall 101, wavelength conversion material layer 102, an emitter 103 with main emission centered around 405 nm wavelength, an emitter 104 with main emission centered around 450 nm wavelength, and a three wire control interface 105.

The emission area comprises (Fig. 5) the first type of LED semiconductor chip 203 emitting at between 385 nm and 430 nm, and having a full width half maximum (FWHM) emission of 5 to 20 nm. The emission area comprises also a second type of LED semiconductor chip 204 emitting at between 430nm and 500 nm, and wavelength conversion material 202 having its peak emission at between 500 nm and 700 nm and having a full width half maximum emission of about 100 nm.

The control interface is having a three wire structure and is to enable independent control of the said two semiconductor chips.

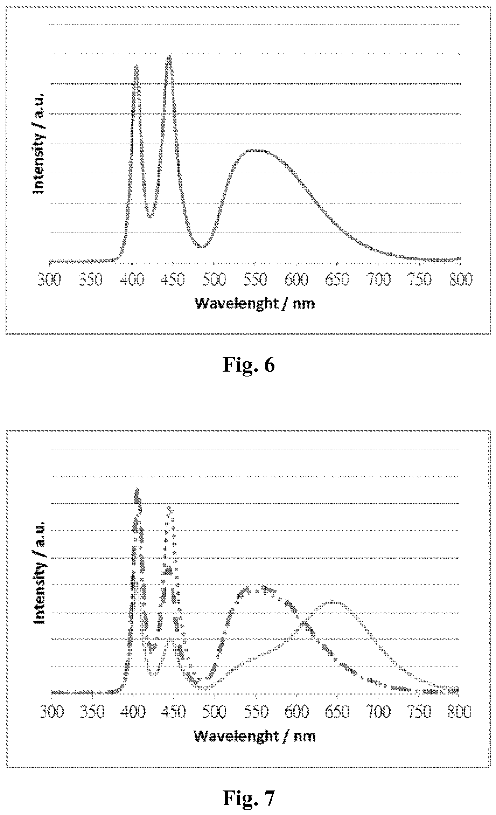

The LED structure emits a spectrum as shown in Figure 6. By controlling the current of the first semiconductor chip emitting at 405 nm, the spectrum can be tuned dynamically as shown in the figure 7 (dotted and dashed lines). Or by changing the wavelength conversion material as shown in the figure 7 (solid line).

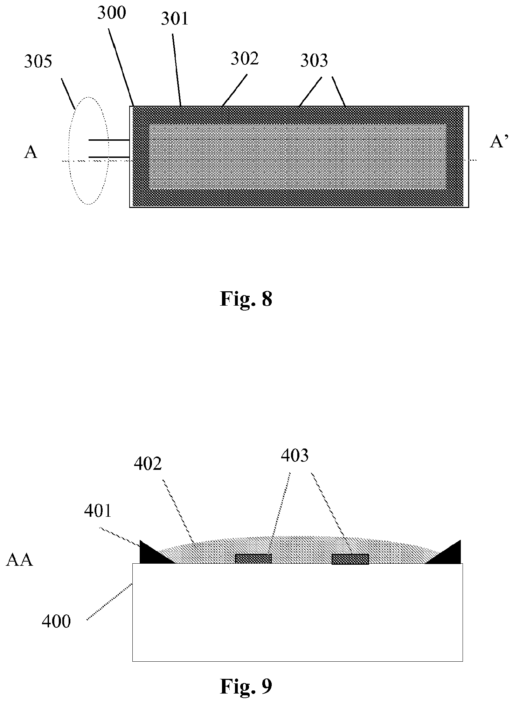

In another embodiment (UV phosphor), not forming part of the present invention as defined by the claims, the LED structure (Fig. 8) is comprised of a substrate 300, an emission area inside the cavity wall 301, wavelength conversion material 302, two emitters 303 with main emission centered between around 365 and 430nm, and a two wire control interface 305.

The emission area comprises (Fig. 9) a single type of LED semiconductor chips 403 emitting at 405 nm, and having a full width half maximum (FWHM) emission of about 14 nm. The emission area also comprises of wavelength conversion material layer 402 having its relatively high extinction coefficient at 405 nm and peak emission between 500 to 700 nm and having a full width half maximum (FWHM) emission normally more than 30 nm The control interface is having a two wire structure and is to enable electrical control of the said emitter chips.

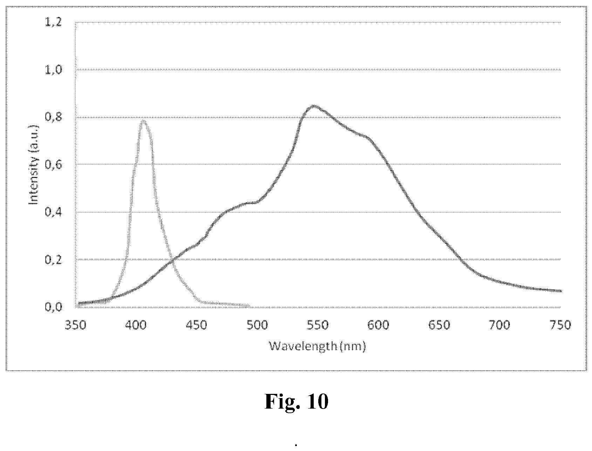

The above LED structure, not forming part of the present invention as defined by the claims, emits a spectrum as shown in Figure 10.

It is to be understood that the embodiments of the invention disclosed are not limited to the particular structures, process steps, or materials disclosed herein, as long as still falling under the scope of the appended claims.

It should also be understood that terminology employed herein is used for the purpose of describing particular embodiments only and is not intended to be limiting.

Reference throughout this specification to "one embodiment of the present invention" or "an embodiment of the present invention" means that a particular feature, structure, or characteristic described in connection with the embodiment is included in at least one embodiment of the present invention as defined by the appended claims.

Furthermore, the described features, structures, or characteristics may be combined in any suitable manner in one or more embodiments, as long as falling under the scope of the present invention as defined by the appended claims. In the above description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. , as long as still falling under the scope of the present invention as defined by the appended claims. In other instances, well-known structures, materials, or operations are not shown or described in detail to avoid obscuring aspects of the invention.

While the forgoing embodiments of the present invention are illustrative of the present invention in one or more particular applications, it will be apparent to those of ordinary skill in the art that numerous modifications in form, usage and details of implementation can be made without the exercise of inventive faculty, and without departing from the scope of the present invention as defined by the appended claims. Accordingly, it is not intended that the invention be limited, except as by the claims set forth below.

### INDUSTRIAL APPLICABILITY

The disclosed use of an integrated LED structure for disinfection has applications for example but not limited to in food production and processing sites, airplanes and hospitals. The ability to have white light illumination and simultaneously having disinfection functionality can greatly reduce infection diseases. Particularly interesting applications are refrigerators at domestic use. In such closed environments low-cost, energy efficient integrated LED structure can be applied in very efficient manner. While the refrigerator's door is closed the emission of short wavelength disinfection emission can be tuned to high intensity and white light can be turned off. Again during anyone opening the door the emission of the short wavelength can be tuned to low-intensity and the white light emission can be turned on.

Another application for continuous disinfection with white light is found with fruit, vegetable, fish and meat desks in grocery stores. Use of continuous disinfection white light in such places would reduce risks of spreading infections while improving the shelf life of fresh products.

In operating rooms in hospitals, as well as in patient rooms and airplanes, white lights with disinfection functionality can be applied to reduce risk for infections.

Another use in greenhouses and food factories is to provide photosynthesis (so call growth) for plants and simultaneously provide germicidal and bactericidal or fungicidal effects for the plants

### REFERENCE SIGNS LIST

- 100: substrate
- 101: cavity wall
- 102: wavelength conversion material layer
- 103,104: emitter
- 105: control interface
- 200: substrate
- 201: cavity wall
- 202: wavelength conversion material layer
- 203,204: semiconductor chip
- 300: substrate
- 301: cavity wall
- 302: wavelength conversion material layer
- 303: emitter
- 305: control interface
- 400: substrate
- 401: cavity wall
- 402: wavelength conversion material layer
- 403: semiconductor chip

### CITATION LIST

### Patent Literature

US 8,398,264 B2
CN 104056289 A
EP 2 554 583 A1
US 2015/0014715 A1
JP 2005-101458 A
US 2009/0224652 A1
US 2012/0043552 A1
EP 2 211 083 A1
US 2009/0323306 A1
WO 2010/053341 A1

### Non-Patent Literature

IEC standard IEC-62471

## Claims

1. Use of a lighting device for disinfection comprising at least one light emitting diode structure comprised of
a substrate (100, 200);
a light emitting area defined on the substrate as a cavity (101, 201);
a first type of light emitting semiconductor source (203) with bactericidal and germicidal characteristics mounted in the cavity (101, 201);
a second type of light emitting semiconductor source (204) mounted in the cavity (101,201) with ability to excite a wavelength conversion material (102, 202) to generate white light; and
the wavelength conversion material (102, 202) formed as a layer on top of the light emitting semiconductor sources (203, 204),
wherein
- in the presence of humans, the first type of light emitting semiconductor source (203) is adjusted to provide white light illumination with a bactericidal and germicidal low-intensity emission in the background of the white light emission, non-perceptible to human eye; and
- in a situation with no humans present, the white light illumination generated by the second type of light emitting semiconductor source (204) exciting the wavelength conversion material (102, 202) is turned off, while the bactericidal and germicidal emission is maximized.

2. The use according to claim 1, wherein the light emitting diode structure further comprises
an electrical circuit layer for connecting the light emitting semiconductor sources (203, 204) to
an electrical control interface (105).

3. The use according to claim 1 or 2, wherein in the light emitting diode structure the first type of light emitting semiconductor source (203) has a peak light emission in the wavelength range of 365 to 430 nm, and the full width half maximum of the emission is below 30 nm and the second type of light emitting semiconductor source (204) has a peak light emission in the wavelength range of 430 to 500 nm.

4. The use according to any of claims 1 to 3, wherein in the light emitting diode structure the first type of light emitting semiconductor source (203) has a peak light emission in the wavelength range of 365 to 430 nm, and the full width half maximum of the emission is below 30 nm, the second type of light emitting semiconductor source (204) has a peak light emission in the wavelength range of 430 to 500 nm, and a third emission peak, of the wavelength conversion material (102, 202), is in the wavelength range of 450 to 750 nm.

5. The use according to any of claims 1 to 3, wherein in the light emitting diode structure the wavelength conversion material (102, 202) has a light emission band in the wavelength range of 450 to 750 nm, with peak luminescence intensity in the wavelength range of 500 to 700 nm and the full width half maximum of the luminescence emission is at least 50 nm, and the material (102, 202) has a maximum normalized extinction coefficient of one in the wavelength range of 430 to 500 nm and a local minimum of normalized extinction coefficient below 0.5 and preferably below 0.3 in the wavelength range of 365 to 430 nm.

6. The use according to any of claims 1 to 5, wherein in the light emitting diode structure color rendering index of visible spectrum is over 70 and color temperature is between 2000K and 8000K, and wherein at least 10% optical power is emitted between 365nm and 430nm wavelength range.

7. The use according to any of claims 1 and 3 to 6, wherein in the light emitting diode structure an electrical circuit layer is formed on the top surface of the substrate (100, 200) for connecting the light emitting semiconductor sources (203, 204) to electrical control interfaces.

8. The use according to any of claims 1 and 3 to 6, wherein in the light emitting diode structure the light emitting semiconductor sources (203, 204) are connected via an electrical circuit layer to an electrical control interface (105) such that the light emitting semiconductor sources can be independently controlled.

## Patentansprüche

1. Verwenden einer Beleuchtungsvorrichtung zur Desinfektion, umfassend mindestens eine Leuchtdiodenstruktur, bestehend aus
einem Substrat (100, 200);
einem lichtemittierenden Bereich, der auf dem Substrat als ein Hohlraum (101, 201) definiert ist;
einer ersten Art von lichtemittierender Halbleiterquelle (203) mit bakteriziden und keimtötenden Eigenschaften, die in dem Hohlraum (101, 201) montiert ist;
einer zweiten Art von lichtemittierender Halbleiterquelle (204), die in dem Hohlraum (101, 201) montiert ist, mit der Fähigkeit, ein Wellenlängenumwandlungsmaterial (102, 202) anzuregen, um weißes Licht zu erzeugen; und
dem Wellenlängenumwandlungsmaterial (102, 202), das als eine Schicht auf einer Oberseite der lichtemittierenden Halbleiterquellen (203, 204) gebildet ist,
wobei
- wobei in der Gegenwart von Menschen die erste Art von lichtemittierender Halbleiterquelle (203) so eingestellt ist, dass sie weißes Licht mit einer bakteriziden und keimtötenden Emission geringer Intensität im Hintergrund der weißen Lichtemission bereitstellt, die für das menschliche Auge nicht wahrnehmbar ist; und
- in einer Situation, in der keine Menschen anwesend sind, die Weißlichtbeleuchtung, die von der zweiten Art von lichtemittierender Halbleiterquelle (204) erzeugt wird, die das Wellenlängenumwandlungsmaterial (102, 202) anregt, ausgeschaltet wird, während die bakterizide und keimtötende Emission maximiert ist.

2. Verwendung nach Anspruch 1, wobei die Leuchtdiodenstruktur weiter
eine elektrische Schaltungsschicht zum Verbinden der lichtemittierenden Halbleiterquellen (203, 204) mit einer elektrischen Steuerschnittstelle (105) umfasst.

3. Verwendung nach Anspruch 1 oder 2, wobei in der Leuchtdiodenstruktur die erste Art von lichtemittierender Halbleiterquelle (203) eine Lichthöchstemission im Wellenlängenbereich von 365 bis 430 nm aufweist und die Halbwertsbreite der Emission unter 30 nm ist und die zweite Art von lichttemittierender Halbleiterquelle (204) eine Lichthöchstemission im Wellenlängenbereich von 430 bis 500 nm aufweist.

4. Verwendung nach einem der Ansprüche 1 bis 3, wobei in der Leuchtdiodenstruktur die erste Art von lichtemittierender Halbleiterquelle (203) eine Lichthöchstemission im Wellenlängenbereich von 365 bis 430 nm aufweist und die Halbwertsbreite der Emission unter 30 nm ist, die zweite Art von lichtemittierender Halbleiterquelle (204) eine Lichthöchstemission im Wellenlängenbereich von 430 bis 500 nm aufweist und eine dritte Höchstemission des Wellenlängenumwandlungsmaterials (102, 202) im Wellenlängenbereich von 450 bis 750 nm ist.

5. Verwendung nach einem der Ansprüche 1 bis 3, wobei in der Leuchtdiodenstruktur das Wellenlängenumwandlungsmaterial (102, 202) ein Lichtemissionsband im Wellenlängenbereich von 450 bis 750 nm aufweist, mit Lumineszenz-Spitzenintensität im Wellenlängenbereich von 500 bis 700 nm und die Halbwertsbreite der Lumineszenzemission mindestens 50 nm ist und das Material (102, 202) einen maximalen normierten Extinktionskoeffizienten von Eins im Wellenlängenbereich von 430 bis 500 nm und ein lokales Minimum des normierten Extinktionskoeffizienten unter 0,5 und bevolrzugt unter 0,3 im Wellenlängenbereich von 365 bis 430 nm aufweist.

6. Verwendung nach einem der Ansprüche 1 bis 5, wobei in der Leuchtdiodenstruktur ein Farbwiedergabeindex des sichtbaren Spektrums über 70 ist und Farbtemperatur zwischen 2000K und 8000K ist und wobei mindestens 10 % optische Leistung in einem Wellenbereich von 365 nm bis 430 nm emittiert wird.

7. Verwendung nach einem der Ansprüche 1 und 3 bis 6, wobei in der Leuchtdiodenstruktur eine elektrische Schaltungsschicht zum Verbinden der lichtemittierenden Halbleiterquellen (203, 204) mit elektrischen Steuerschnittstellen an der oberen Oberfläche des Substrats (100, 200) gebildet ist.

8. Verwendung nach einem der Ansprüche 1 und 3 bis 6, wobei in der Leuchtdiodenstruktur die lichtemittierenden Halbleiterquellen (203, 204) über eine elektrische Schaltungsschicht mit einer elektrischen Steuerschnittstelle (105) verbunden sind, sodass die lichtemittierenden Halbleiterquellen unabhängig gesteuert werden können.

## Revendications

1. Utilisation d'un dispositif d'éclairage pour une désinfection comprenant au moins une structure de diode électroluminescente composée de
un substrat (100, 200) ;
une zone électroluminescente définie sur le substrat sous la forme d'une cavité (101, 201) ;
un premier type de source électroluminescente à semi-conducteur (203) présentant des caractéristiques bactéricides et germicides montée dans la cavité (101, 201) ;
un second type de source électroluminescente à semi-conducteur (204) montée dans la cavité (101, 201) avec la capacité d'exciter un matériau de conversion de longueur d'onde (102, 202) pour générer une lumière blanche ; et
le matériau de conversion de longueur d'onde (102, 202) façonné sous la forme d'une couche sur la partie supérieure des sources électroluminescentes à semi-conducteur (203, 204),
dans laquelle
- en présence d'êtres humains, le premier type de source électroluminescente à semi-conducteur (203) est réglé pour fournir un éclairage en lumière blanche avec une émission bactéricide et germicide de faible intensité en arrière-plan de l'émission de lumière blanche, non perceptible à l'œil humain ; et
- dans une situation dans laquelle aucun être humain n'est présent, l'éclairage en lumière blanche généré par le second type de source électroluminescente à semi-conducteur (204) excitant le matériau de conversion de longueur d'onde (102, 202) est éteint, en même temps que l'émission bactéricide et germicide est augmentée au maximum.

2. Utilisation selon la revendication 1, dans laquelle la structure de diode électroluminescente comprend en outre
une couche de circuit électrique pour connecter les sources électroluminescentes à semi-conducteur (203, 204) à une interface de commande électrique (105).

3. Utilisation selon la revendication 1 ou 2, dans laquelle, dans la structure de diode électroluminescente, le premier type de source électroluminescente à semi-conducteur (203) présente une émission de lumière maximale dans la plage de longueur d'onde de 365 à 430 nm et la largeur à mi-hauteur de l'émission est inférieure à 30 nm et le second type de source électroluminescente à semi-conducteur (204) présente une émission de lumière maximale dans la plage de longueur d'onde de 430 à 500 nm.

4. Utilisation selon l'une quelconque des revendications 1 à 3, dans laquelle, dans la structure de diode électroluminescente, le premier type de source électroluminescente à semi-conducteur (203) présente une émission de lumière maximale dans la plage de longueur d'onde de 365 à 430 nm et la largeur à mi-hauteur de l'émission est inférieure à 30 nm, le second type de source électroluminescente à semi-conducteur (204) présente une émission de lumière maximale dans la plage de longueur d'onde de 430 à 500 nm et un troisième pic d'émission du matériau de conversion de longueur d'onde (102, 202) est dans la plage de longueur d'onde de 450 à 750 nm.

5. Utilisation selon l'une quelconque des revendications 1 à 3, dans laquelle, dans la structure de diode électroluminescente, le matériau de conversion de longueur d'onde (102, 202) présente une bande d'émission de lumière dans la plage de longueur d'onde de 450 à 750 nm, avec une intensité de luminescence maximale dans la plage de longueur d'onde de 500 à 700 nm et la largeur à mi-hauteur de l'émission de luminescence est au moins 50 nm, et le matériau (102, 202) présente un coefficient d'extinction normalisé maximal de un dans la plage de longueur d'onde de 430 à 500 nm et un minimum local de coefficient d'extinction normalisé inférieur à 0,5 et, de préférence, inférieur à 0,3 dans la plage de longueur d'onde de 365 à 430 nm.

6. Utilisation selon l'une quelconque des revendications 1 à 5, dans laquelle, dans la structure de diode luminescente, un indice de rendu de couleurs d'un spectre visible dépasse 70 et une température de couleur est comprise entre 2 000 K et 8 000 K et dans laquelle une puissance optique d'au moins 10 % est émise dans une plage de longueur d'onde comprise entre 365 nm et 430 nm.

7. Utilisation selon l'une quelconque des revendications 1 et 3 à 6, dans laquelle, dans la structure de diode luminescente, une couche de circuit électrique est formée sur la surface supérieure du substrat (100, 200) pour connecter les sources électroluminescentes à semi-conducteur (203, 204) à des interfaces de commande électrique.

8. Utilisation selon l'une quelconque des revendications 1 et 3 à 6, dans laquelle, dans la structure de diode luminescente, les sources électroluminescentes à semi-conducteur (203, 204) sont connectées par le biais d'une couche de circuit électrique à une interface de commande électrique (105) de telle sorte que les sources électroluminescentes à semi-conducteur puissent être commandées séparément.
